(19) Europäisches Patentamt
European Patent Office
Office européen des brevets

(11) EP 4 656 130 A1

(12) EUROPEAN PATENT APPLICATION
published in accordance with Art. 153(4) EPC

(43) Date of publication:
03.12.2025  Bulletin 2025/49

(51) International Patent Classification (IPC):
A61B 5/02 (2006.01)     A61B 7/02 (2006.01)
G16H 50/20 (2018.01)

(21) Application number: 23923066.7

(22) Date of filing: 14.12.2023

(52) Cooperative Patent Classification (CPC):
A61B 5/02; A61B 7/02; G16H 50/20

(86) International application number:
PCT/KR2023/020660

(87) International publication number:
WO 2024/172267 (22.08.2024 Gazette 2024/34)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 17.02.2023  KR 20230021404

(71) Applicants:
• The Asan Foundation
Seoul 05505 (KR)
• UNIVERSITY OF ULSAN FOUNDATION FOR
INDUSTRY COOPERATION
Nam-gu
Ulsan 44610 (KR)

(72) Inventors:
• KIM, Sung Hoon
Seoul 05556 (KR)
• SEO, Woo Young
Seoul 05544 (KR)
• PARK, Seong Yong
Seoul 05537 (KR)
• KIM, Hyun Seok
Seoul 02724 (KR)
• KIM, Jin Young
Uiwang-si, Gyeonggi-do 16083 (KR)

(74) Representative: dompatent
Partnerschaft von
Patentanwälten und Rechtsanwälten mbB
Deichmannhaus am Dom
Bahnhofsvorplatz 1
50667 Köln (DE)

(54) METHOD FOR MEASURING PRELOAD OF GENERAL ANESTHESIA SURGERY PATIENT ON BASIS OF ACOUSTIC VARIABILITY INDEX, AND ELECTRONIC DEVICE FOR PERFORMING SAME

(57) Disclosed is a method for analyzing a heart sound by an electronic device for measuring a preload of a general anesthesia surgery patient including splitting a heart sound and a breathing sound from heart-lung sound data of a patient obtained externally, specifying a breathing section from the split breathing sound, obtaining a heart sound envelope for obtaining a peak signal by converting a heart sound signal, obtaining a heart sound index from the heart sound envelope, and calculating an acoustic variability index (AVI) by analyzing the heart sound index.

[FIG. 2]

**Description**

[TECHNICAL FIELD]

Embodiments of the present disclosure described herein relate to a method for measuring the preload of a general anesthesia surgery patient, and more particularly, relate to a method, a device, and a computer program for measuring a preload by noninvasively determining a patient's fluid responsiveness based on the patient's heart-lung sound, as a method for measuring the preload of a general anesthesia surgery patient based on an acoustic variability index, and an electronic device for performing the same.

[BACKGROUND ART]

A surgery patient may experience rapid fluctuations in blood volume in a short period of time due to bleeding. Accordingly, in most surgeries where blood volume fluctuations are required, the blood volume fluctuations are estimated by indirect measurement of a preload, which is the pressure when the ventricles are filled with blood at the end of diastole.

Swan-Ganz catheter, which is connected directly to the jugular vein, right atrium, right ventricle, and pulmonary artery, may be used to directly measure the patient's cardiac output (CO) and stroke volume (SV), but it should only be used in a limited number of patients due to the possibility for potentially fatal complications such as arrhythmias, pulmonary infarction, pulmonary artery rupture, and infection.

In conventional clinical practice, a method of monitoring the preload by directly measuring Pulse Pressure Variation (PPV) from continuous Arterial Blood Pressure (ABP) measured from an arterial line catheter or by estimating Stroke Volume Variation (SVV) from arterial pressure calculations, and a method of calculating a Pleth Variability index (PVi) by calculating blood pressure waveform data are used.

However, the arterial line catheter for measuring PPV and SVV among the indices is invasive testing methods, and thus it is still a heavy burden on the patient's body. The PVi is non-invasive but relatively inaccurate, and thus a method of measuring a preload by using a new index is required to be developed.

[DETAILED DESCRIPTION OF THE INVENTION]

[TECHINICAL PROBLEM]

Embodiments of the present disclosure provide a method for noninvasively measuring a preload by analyzing heart-lung sound signals and calculating an acoustic variability index (AVI).

Embodiments of the present disclosure provide a general anesthesia surgery patient preload measurement method based on acoustic variability index and an electronic device executing the same.

Problems to be solved by the present disclosure are not limited to the problems mentioned above, and other problems not mentioned will be apparent by those skilled in the art from the following description.

[TECHNICAL SOLUTION]

According to an embodiment, a method for analyzing a heart sound by an electronic device for measuring a preload of a general anesthesia surgery patient includes splitting a heart sound and a breathing sound from heart-lung sound data of a patient obtained externally, specifying a breathing section from the split breathing sound, obtaining a heart sound envelope for obtaining a peak signal by converting a heart sound signal, obtaining a heart sound index from the heart sound envelope, and calculating an acoustic variability index (AVI) by analyzing the heart sound index.

According to an embodiment, a method for analyzing a heart sound by an electronic device for measuring a preload of a general anesthesia surgery patient includes splitting a heart sound and a breathing sound from heart-lung sound data of a patient obtained externally, obtaining an envelope of a waveform generated as a result of performing a Hilbert transform on the heart sound, detecting a first heart sound and a second heart sound, calculating at least one of a peak-specific location, amplitude, and an area of the waveform, detecting a breathing cycle from the breathing sound, specifying a breathing section from the breathing cycle, calculating a heart sound index for the respective breathing section, and calculating an AVI by analyzing the heart sound index.

According to an embodiment, an electronic device for measuring a preload of a general anesthesia surgery patient includes a memory that stores at least one instruction and a processor that performs a preload measurement function by executing the instruction. The processor splits a heart sound of the patient through a high-pass filter and a band-pass filter by using a heart-lung sound split module, specifies a section between inhalation and expiratory of the patient by using an envelope of a waveform generated through a Hilbert transform by using a breathing section specifying module, detects a first heart sound signal and a second heart sound signal corresponding to local maxima of the envelope by using a heart sound index calculation module, and calculates an acoustic variability index from at least one of a time variation, an amplitude variation, and an area variation of each of the first heart sound signal and the second heart sound signal by using an acoustic variability index computation module.

In addition, a computer-readable recording medium for recording a computer program for performing the method for implementing the present disclosure may be further provided.

[ADVANTAGEOUS EFFECTS OF THE INVENTION]

According to an embodiment of the present disclosure, a method for measuring the preload of a general anesthesia surgery patient and an electronic device executing the same may noninvasively measure a preload of a patient by acquiring and analyzing heart-lung sounds.

According to an embodiment of the present disclosure, a method for measuring the preload of a general anesthesia surgery patient and an electronic device executing the same may measure the preload by utilizing heart-lung sound capable of being obtained from an esophageal stethoscope because an esophageal stethoscope is essential for general anesthesia surgery, thereby improving patient safety compared to conventional invasive methods (Swan-Ganz catheter, arterial pressure catheter) and improving accuracy compared to conventional noninvasive methods (blood volume variation).

According to an embodiment of the present disclosure, a method for measuring the preload of a general anesthesia surgery patient and an electronic device executing the same may specify a plurality of heart sounds S1 and S2 and may automatically detect their indices by using only heart-lung sound signals without an ECG signal.

Effects of the present disclosure are not limited to the effects mentioned above, and other effects not mentioned will be apparent by those skilled in the art from the following description.

[DESCRIPTION OF THE DRAWINGS]

FIG. 1 is a block diagram illustrating a heart-lung sound analysis system, according to an embodiment of the present disclosure.

FIG. 2 is a flowchart illustrating a method for measuring a general anesthesia surgery patient preload based on acoustic variability index, according to an embodiment of the present disclosure.

FIG. 3 is a waveform diagram showing that a heart sound is split from a heart-lung sound and transformed into an envelope form for analysis by a preload measurement device, according to an embodiment of the present disclosure.

FIG. 4 is a diagram illustrating that a preload measurement device according to an embodiment of the present disclosure splits a heart sound and a lung sound from a heart-lung sound through a band-pass filter.

FIG. 5 is a diagram for describing a method in which a preload measurement device according to an embodiment of the present disclosure determines a breathing cycle based on a peak.

FIG. 6 is a diagram showing a method, in which a preload measurement device according to an embodiment of the present disclosure obtains a heart sound index.

FIGS. 7A and 7B are diagrams illustrating that a preload measurement device according to an embodiment of the present disclosure corrects a first heart sound split phenomenon.

FIG. 8 is a graph showing a comparison between amplitude of a second heart sound and SVR among heart sound indices obtained according to an embodiment of the present disclosure.

FIG. 9 is a graph showing a comparison between an amplitude change of a first heart sound and SVV among the heart sound indices obtained according to an embodiment of the present disclosure.

FIG. 10 is a graph showing a comparison between an acoustic variability index derived according to an embodiment of the present disclosure and SVV, which is one of existing utilized indices.

FIG. 11 is a block diagram illustrating an example of a preload measurement device, according to an embodiment of the present disclosure.

[BEST MODE]

The same reference numerals denote the same elements throughout the present disclosure. The present disclosure does not describe all elements of embodiments. Well-known content in a technical field, to which the present disclosure belongs, or redundant content in which embodiments are the same as one another will be omitted. A term such as 'unit, module, member, or block' used in the specification may be implemented with software or hardware. According to embodiments, a plurality of 'units, modules, members, or blocks' may be implemented with one component, or a single 'unit, module, member, or block' may include a plurality of components.

Throughout this specification, when it is supposed that a portion is "connected" to another portion, this includes not only a direct connection, but also an indirect connection. The indirect connection includes being connected through a wireless communication network.

Furthermore, when a portion "comprises" a component, it will be understood that it may further include another component, without excluding other components unless specifically stated otherwise.

Throughout this specification, when it is supposed that a member is located on another member "on", this includes not only the case where one member is in contact with another member but also the case where another member is present between two other members.

Terms such as 'first', 'second', and the like are used to distinguish one component from another component, and thus the component is not limited by the terms described above.

Unless there are obvious exceptions in the context, a singular form includes a plural form.

In each step, an identification code is used for convenience of description. The identification code does not describe the order of each step. Unless the context clearly states a specific order, each step may be performed differently from the specified order.

Hereinafter, operating principles and embodiments of the present disclosure will be described with reference to the accompanying drawings.

In this specification, a 'preload measurement device' may mean a general anesthesia surgery patient preload measurement method based on acoustic variability index and an electronic device executing the same. In the present disclosure, a 'preload measurement device' includes all various devices capable of providing results to a user by performing arithmetic processing. For example, the preload measurement device according to an embodiment of the present disclosure may include all of a computer, a server device, and a portable terminal, or may be in any one form.

Here, for example, the computer may include a notebook computer, a desktop computer, a laptop computer, a tablet PC, a slate PC, and the like, which are equipped with a web browser.

A server device may be a server that processes information by communicating with an external device and may include an application server, a computing server, a database server, a file server, a game server, a mail server, a proxy server, and a web server.

For example, the portable terminal may be a wireless communication device that guarantees portability and mobility, and may include all kinds of handheld-based wireless communication devices such as a smartphone, a personal communication system (PCS), a global system for mobile communication (GSM), a personal digital cellular (PDC), a personal handyphone system (PHS), a personal digital assistant (PDA), International Mobile Telecommunication (IMT)-2000, a code division multiple access (CDMA)-2000, W-Code Division Multiple Access (W-CDMA), and Wireless Broadband Internet terminal (Wibro) terminal, and a wearable device such as a timepiece, a ring, a bracelet, an anklet, a necklace, glasses, a contact lens, or a head-mounted device (HMD).

The treatment of anesthetized patients or critically ill patients requires monitoring of a large amount of data. A patient's electrocardiogram, blood pressure, end-expiratory carbon dioxide pressure, peripheral oxygen saturation, and body temperature may be generally monitored by using commonly used monitoring devices currently. Commonly unmonitored but clinically important data includes total blood amount (body fluid content) (hereinafter referred to as "preload" being the same/similar meaning) and cardiac contractile force.

The preload is also referred to as a "reserve load" and is the amount of blood that fills the ventricles at the end of diastole of the heart. As the preload increases, cardiac output increases in a normal heart.

Injury patients or surgery patients may experience rapid changes in blood volume in a short period of time due to reasons such as bleeding. In such cases, it is necessary to know the preload to detect the presence or absence of such changes. That is, appropriate measures such as blood transfusion or intravenous fluid infusion may be taken by accurately identifying the preload in real time, and thus the preload may be evaluated as data directly related to the patient's life. The preload may be indirectly estimated after basic monitoring data such as blood pressure or oxygen saturation is obtained by using conventional monitoring devices. However, as blood pressure may be normal until significant blood loss has occurred, the preload lacks accuracy and real-time monitoring is difficult by using the preload.

Another method of evaluating the preload is "fluid responsiveness". The fluid responsiveness is based on the idea that when a patient's blood volume is insufficient while the patient is administered fluid, there is responsiveness that blood pressure increases. It may be interpreted that there is responsiveness (response(+)) (i.e., the preload is low) when the blood pressure increases after fluid is administered, or the preload is sufficient when there is no response (response(-)). The fluid responsiveness is a well-known method of evaluating whether a patient's blood volume is adequate. This is a concept designed to predict before administration because there are situations where the administration of fluid itself is harmful to a patient. There is currently no known index that perfectly predicts the fluid responsiveness, but many monitor devices released are being developed to display indices indicating the fluid responsiveness on a screen.

The cardiac contractile force (Contractility) refers to the strength of contraction and relaxation of the cardiac muscle. According to 'Frank-Starling law', it may be defined as the relationship of cardiac output according to left ventricular end-diastolic pressure (LVEDP). In many cases, when blood pressure drops even though the preload is sufficient, the reason is that cardiac contractile force is insufficient. Nowadays, there is no equipment other than echocardiography to evaluate the cardiac contractile force. However, the echocardiography equipment requires expertise and continuous monitoring is difficult, making it difficult to use conveniently in actual clinical settings.

Representative examples of data monitoring devices currently available on the market to monitor anesthetized patients or critically ill patients are as follows.

Vigilence® device from Edward Lifescience® inserts a catheter into the pulmonary artery and measures various hemodynamic indices by using a hemodilution method. In particular, it evaluates the patient's condition by observing whether cardiac output (CO) increases by 10 to 15% or more due to fluid administration. This uses the index of the above-described fluid responsiveness, but as mentioned above, there may be a delay error (time delay) due to the body heat dilution time. Accordingly, the accuracy is low in cases of rapid hemodynamic changes, and the risk is high, because a long catheter tube penetrates a heart and is placed in a pulmonary artery.

Vigileo® device, which is another device from Edward Lifescience®, obtains data from the shape of the arterial pressure waveform measured from a radial artery and calculates Stroke Volume Variation (SVV). There are reports that it has the disadvantage of requiring invasive arterial cannulation and that there is a large error rate in situations requiring administration of large amounts of pressor agents, such as in sepsis.

NICOM® device from Chita Medical® estimates total blood amount based on changes in thoracic impedance measured by attaching electrical electrodes to four corners of a thoracic cage. The present device is too inaccurate, and a signal is interfered with by the use of electrocautery during surgery.

In addition, equipment for echocardiographic measurements is manufactured by Philips®, GE®, CardioQ®, and Zonare®, but continuous monitoring is difficult and skill is essential, and thus there are limitations in providing data necessary for anesthetized patients or critically ill patients.

For example, the conventional equipment uses invasive methods to increase accuracy. However, some equipment that is not invasive has low accuracy, requires skill, and is difficult to monitor continuously.

Accordingly, the development of equipment that provides preload and/or cardiac contractile force for surgery patients or severely injury patients is required. In particular, the development of equipment that may provide data regardless of the skill level of an operator while increasing accuracy and having excellent sustainability by using non-invasive methods is required.

Studies have shown that the signal extractable from heart sounds in the prior art is significant. However, they have not been defined in a form capable of being used in actual surgery, and the calculation of the heart sound index has not been specified. According to existing papers on heart sound detection, real-time automatic detection requires an additional ECG signal, and real-time automatic detection of a heart sound is impossible with only a heart-lung sound signal.

Accordingly, the present disclosure may specify a first heart sound S1 and a second heart sound S2 through only a heart-lung sound signal without an ECG signal and may automatically detect the index. In addition, the present disclosure proposes a new index called acoustic variability index (AVI) capable of utilizing a heart sound.

Hereinafter, medical terms are defined for convenience of description.

ECG: electrocardiogram
SVV; stroke volume variation
HS; heart sound
PCG; phonocardiogram
PP; pulse pressure (mmHg)
STI (S1-S2 Interval); systolic time interval (ms)
S1, S2; first heart sound, second heart sound

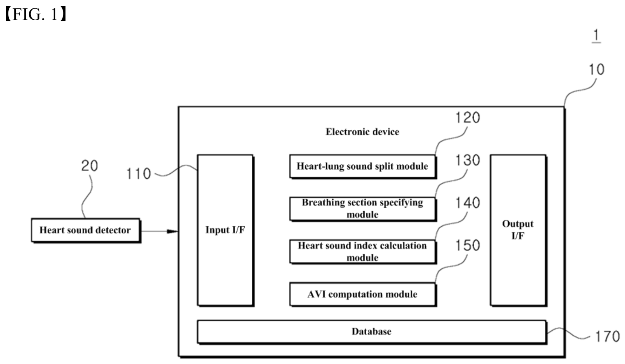

FIG. 1 is a block diagram illustrating a heart-lung sound analysis system 1, according to an embodiment of the present disclosure.

A preload of a general anesthesia surgery patient is an important index for determining the patient's cardiac output. There is a monitoring device (Vigillance from Edwards Lifesciences) that uses a Swan-Ganz catheter that may directly measure cardiac output. However, this is very burdensome to a patient as it requires direct insertion of a catheter into the heart, and thus it is only used in very limited situations. A monitoring device EV1000 (Edwards Lifesciences) that indirectly measures the preload by using SVV is less invasive than Swan-Ganz catheter, but is also requires an 'arterial pressure catheter' directly into the patient's artery. Because the use of the catheter carries the potential for complications, whether to use it is decided by a clinician. Among 66,000 non-cardiac surgery patients at a single tertiary care facility, 'arterial pressure catheters' were used in only around 30% of operations (Jungyo Suh & SangWook Lee, Preoperative prediction of the need for arterial and central venous catheterization using machine learning techniques, Scientific reports, 2022).

Noninvasive preload measurement methods include Pleth Variability Index (PVi) (Masimo) using a plethysmograph, but the accuracy of fluid responsiveness is generally lower than that of SVV (HTA-2014-31 Plethysmography Variability Index Measurement Method).

In the meantime, an esophageal stethoscope is used to monitor body temperature and listen to heart-lung sounds during general anesthesia surgery, according to the recommendations of the American Society of Anesthesiologists (ASA) and Health Insurance Review & Assessment Service. Although a heart sound signal included in the heart-lung sound is an important tool that provides information about the patient's heart condition, it is currently rarely used.

Referring to FIG. 1, the heart-lung sound analysis system 1 may include a preload measurement device 10 and a heart sound detector 20.

The heart-lung sound analysis system 1 may obtain the heart-lung sound of a general anesthesia patient, may split a heart sound and a lung sound from the obtained medical information (heart-lung sound), may extract features necessary for surgery and patient management, and may process the features into data necessary for surgery or procedures.

It is assumed that the preload measurement device 10 is an electronic device that implements a general anesthesia surgery patient preload measurement method based on an acoustic variability index, and the general anesthesia surgery patient preload measurement method based on an acoustic variability index is implemented through the preload measurement device 10. According to an embodiment, the preload measurement device 10 may be referred to as an "electronic device".

The heart sound detector 20 may be a stethoscope or a microphone. The heart sound detector 20 may also be inserted into the patient's body by using separate equipment. In an embodiment, the heart sound detector 20 may be a catheter-type esophageal stethoscope (P) that has been previously inserted and positioned in the esophagus of a patient. In an embodiment, the heart sound detector 20 may be mounted on an esophageal stethoscope in the form of a miniature microphone. In an embodiment, the heart sound detector 20 may be attached to the outer end of a heart sound stethoscope or any other location in the middle, and there is no limitation on the attachment range within a range in which heart-lung sounds may be detected.

According to an embodiment of the present disclosure, the heart-lung sound analysis system 1 may measure preload by detecting heart sound peak values by using a Hilbert transform and a Hilbert envelope for the heart sound based on an acoustic variability index (AVI). The method of measuring the preload based on the acoustic variability index (AVI) will be described in more detail with reference to the drawings below.

The preload measurement device 10 may include an input interface (I/F)(110), a heart-lung sound split module 120, a breathing section specifying module 130, a heart sound index calculation module 140, an AVI computation module 150, an output I/F 160, and a database 170. The preload measurement device 10 of FIG. 1 is only an embodiment of the present disclosure, and thus the present disclosure is not limited to an embodiment of FIG. 1.

The input I/F 110 may be used to enter image information (or signal), audio information (or signal), data, or information entered by a user, and may include at least one of at least one camera, at least one microphone, and the user input I/F 110. Speech data or image data collected by the input I/F 110 may be analyzed and processed as a control command of a user.

In an embodiment of the present disclosure, the input I/F 110 serves as a passage for various types of external devices connected to the present device. The interface unit may include at least one of a wired/wireless headset port, an external charger port, a wired/wireless data port, a memory card port, a port for connecting a device equipped with a subscriber identification module (SIM), an audio input/output (I/O) port, a video I/O port, and an earphone port. In the present device, appropriate control related to an external device connected to the interface unit may be performed.

The user input I/F 110 may be used to receive information from a user. When information is entered through the user input I/F 110, a control unit may control operations of the present device to correspond to the input information. This user input I/F 110 may include a hardware-type physical key (e.g., a button, a dome switch, a jog wheel, or a jog switch that is located on at least one of the front, back, and sides of the present device) and a software-type touch key. For example, the touch key may consist of a virtual key, a soft key, or a visual key displayed on a touch screen-type display unit through software processing or may consist of a touch key positioned on a portion other than the touch screen. In the meantime, the virtual key or the visual key may be displayed on the touch screen while having various shapes. For example, the virtual key or the visual key may be formed of graphics, texts, icons, video, or a combination thereof.

The sensor unit senses at least one of information about interiors of the present device, surrounding environmental information surrounding the present device, and user information and generates a sensing signal corresponding to the sensed result. On the basis of the sensing signal, the control unit may control the driving or operation of the present device or may perform the data processing, function, or operation associated with an application program installed in the present device.

The sensor unit may include at least one of a proximity sensor, an illumination sensor, a touch sensor, an acceleration sensor, a magnetic sensor, a G-sensor, a gyroscope sensor, a motion sensor, an RGB sensor, an infrared sensor (IR sensor), a finger scan sensor, an ultrasonic sensor, an optical sensor (e.g., a camera), a microphone, an environmental sensor (e.g., including at least one of a barometer, a hygrometer, a thermometer, a radiation detection sensor, a heat detection sensor, and a gas detection sensor), and a chemical sensor (e.g., a healthcare sensor, a biometric sensor, or the like). In the meantime, the present device may combine and utilize pieces of information sensed by at least two or more of these sensors.

According to an embodiment of the present disclosure, the input I/F 110 may receive a heart-lung sound of a patient from an external device. For example, the input I/F 110 may receive the patient's heart-lung sound through the heart sound detector 20.

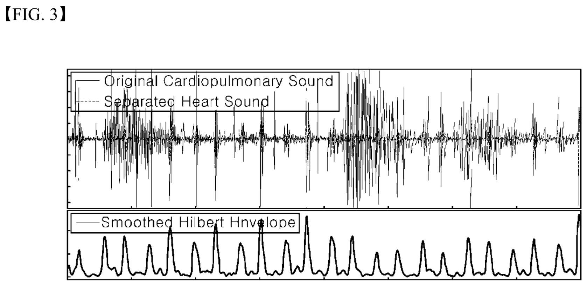

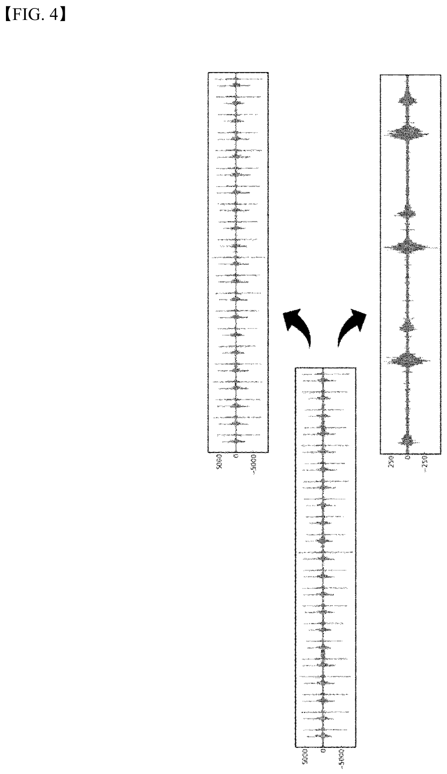

The heart-lung sound split module 120 may split a heart sound and a lung sound from the heart-lung sound. The operation of the heart-lung sound split module 120 will be described in more detail with reference to FIGS. 3 and 4.

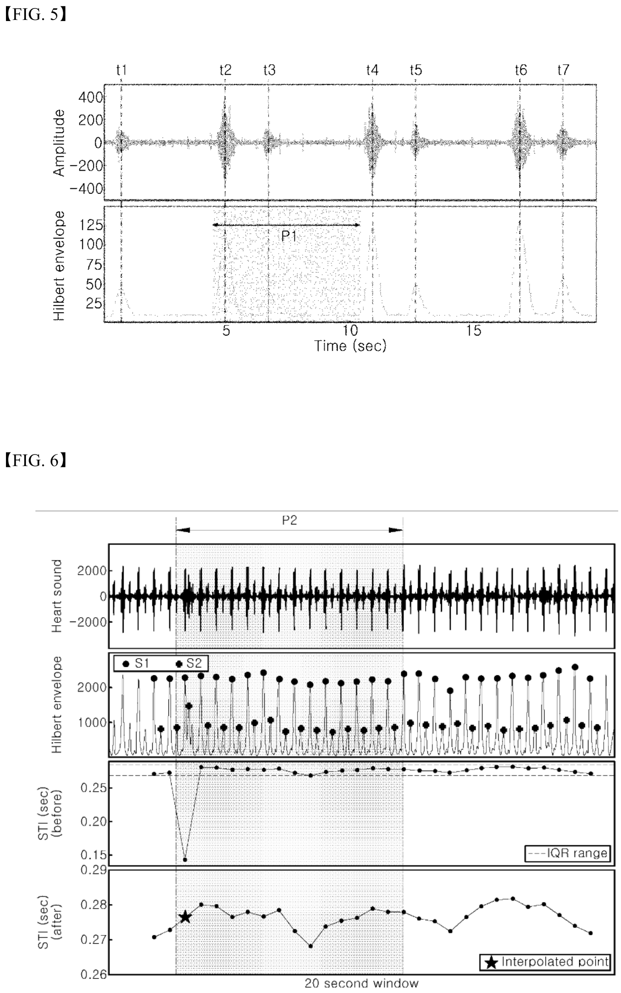

The breathing section specifying module 130 may determine an inhalation time point and an expiratory time point from the lung sound, and may specify a section between inhalation and exhalation as the breathing section by specifying an inflection point as the inhalation start point based on primary differentiation. The operation of the breathing section specifying module 130 will be described in more detail with reference to FIG. 5.

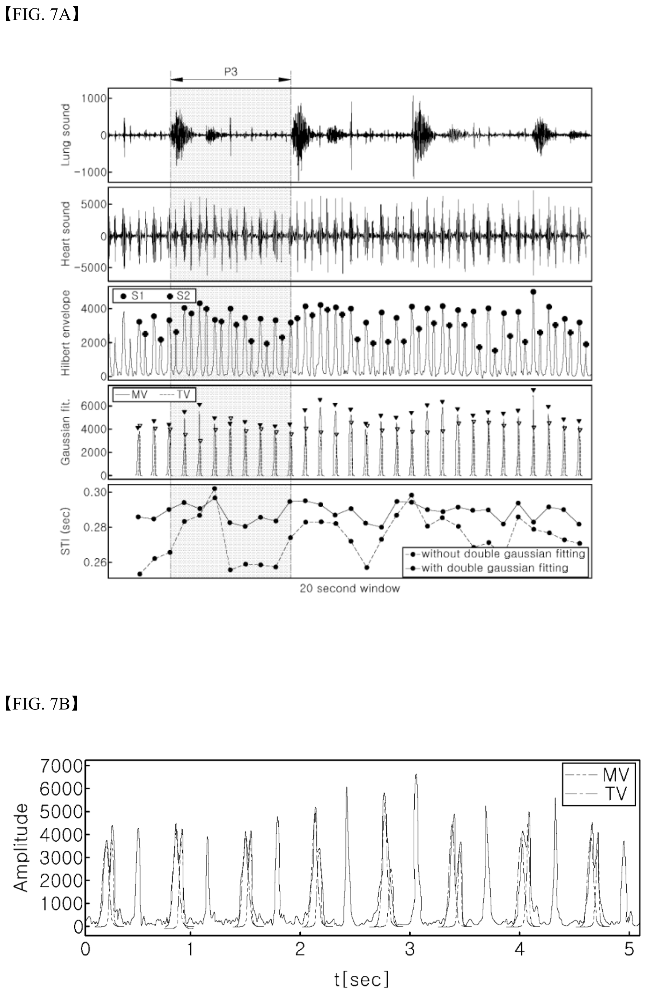

The heart sound index calculation module 140 may determine the first heart sound S1 and the second heart sound S2 from the heart sound, and may calculate the heart sound index related to the peak amplitude, area, or time of each of the first heart sound S1 and the second heart sound S2. The operation of the heart sound index calculation module 140 will be described in more detail with reference to FIGS. 6, 7A, and 7B.

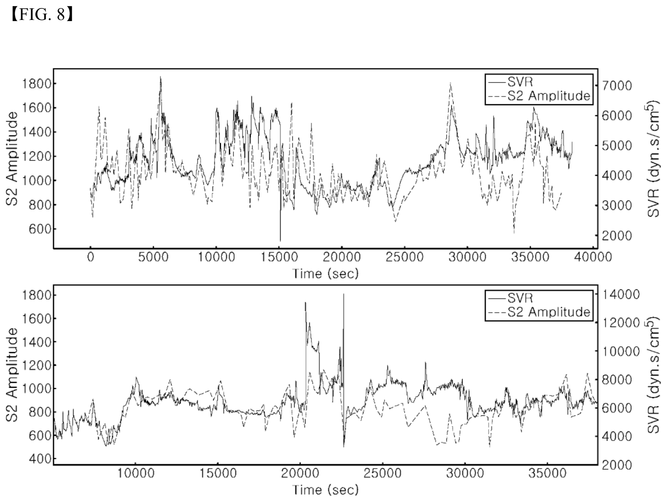

The AVI computation module 150 may generate data for measuring preload. The AVI computation module 150 will be described in more detail with reference to FIG. 8.

The processor is configured to control overall organic operations of various functional units of an electronic device 100, such as processing one or more commands required for controlling the preload measurement device 10, performing operations according to the commands, and making determination according to program logic. The processor may provide or process appropriate information or functions to a user, by processing signals, data, information, or the like, which is input or output through the input/output I/F 110 or the various processing modules 120 to 150, or driving an application program stored in the database 170. The processed data may be stored in the memory, may be used to build the database 170, or may be transmitted to the outside through the input/output I/F 110 or the various processing modules 120 to 150. The processor may be implemented with a general-purpose processor, a special-purpose processor, or an application processor. In an embodiment, the processor may be implemented as an operation processor (e.g., a central processing unit (CPU), a graphic processing unit (GPU), an application processor (AP), and the like) including dedicated logic circuits (e.g., a field programmable gate array (FPGA), an application specific integrated circuits (ASICs), and the like), but is not limited thereto. In an embodiment, it is not excluded that the processor is implemented as a digital signal processor (DSP) that performs high-speed processing by converting analog signals into digital signals, a micro controller unit (MCU), or a neural processing unit (NPU) specialized in processing an artificial neural network.

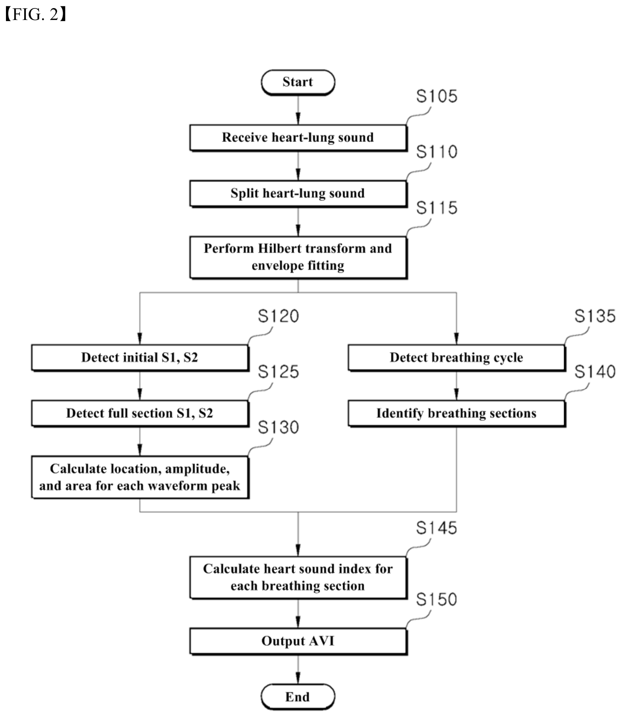

Furthermore, the processor may control one of the components described above or the combination of the components to implement various embodiments of the present disclosure described below with reference to FIGS. 2 to 11 on the present device.

The output I/F 160 may include at least one of a display unit, a sound output interface, a haptic module, and an optical output interface, which are used to generate an output associated with visual, auditory, or tactile sensation. The display unit may have a mutual layer structure with a touch sensor or may be integrally formed with the touch sensor, thereby implementing the touch screen. Such the touch screen may provide an output interface between the present device and a user as well as operating as a user input unit that provides an input interface between the present device and the user.

The display unit displays (outputs) information processed by the present device. For example, the display unit may display execution screen information of an application program (e.g., an application) running on the present device, or a user interface (UI) or graphical user interface (GUI) information according to such the execution screen information.

The sound output interface may output audio data, which is received through a communication unit or stored in the memory, or may output audio signals related to functions performed by the present device. The sound output I/F 160 may include a receiver, a speaker, a buzzer, and the like.

The haptic module generates various tactile effects that the user is capable of feeling. A representative example of the tactile effect generated by the haptic module is vibration. The amplitude and pattern of the vibration generated from the haptic module may be controlled by the user's selection or the settings of a control unit. Furthermore, in addition to the vibration, the haptic module may generate various tactile effects such as an effect of stimulation of a pin arrangement that vertically moves on a contact skin surface, blowing force or suction force of air through a nozzle or suction port, rubbing against a skin surface, a contact of an electrode, electrostatic force, or the like, or an effect by the coolness and warmth generated by using elements capable of absorbing heat or generating heat.

The haptic module may not only deliver a tactile effect through a direct contact, and may but also be implemented such that the user is capable of feeling a tactile effect through a muscle sense of a finger or arm. The two or more haptic modules may be provided depending on a configuration aspect of the present device.

The optical output I/F 160 may output a signal for providing a notification that an event occurs, by using light from a light source of the present device, or may output media such as images to be displayed on an external screen by using a projector. In an embodiment, the optical output I/F 160 may adjust the wavelength, magnitude, and projection location of light such that an image is projected three-dimensionally onto an object to express the image in augmented reality.

The output I/F 160 serves as a passage for various types of external devices connected to the present device. The interface unit may include at least one of a wired/wireless headset port, an external charger port, a wired/wireless data port, a memory card port, a port for connecting a device equipped with a subscriber identification module (SIM), an audio input/output (I/O) port, a video I/O port, and an earphone port.

The database 170 refers to a set of data that is collectively managed for the purpose of being shared and used by storing various types of information. The database 170 may store data temporarily or semi-permanently. For example, the database 170 may store an operating system (OS) for operating at least one device, data for hosting a website, or data regarding an application (e.g., a web application). Moreover, the database may store modules in the form of computer code as described above. The database 170 is managed through middleware separate from an application program.

The database 170 includes a relational database (RDB), a key-value database, an object database, a document database, a memory database, and the like.

The database 170 may store data for supporting various functions of the apparatus, and a program for operations of the control unit, may store pieces of input/output data (e.g., music files, still images, videos, and the like), and may store a plurality of application programs (or applications) running on the apparatus, pieces of data for operations of the present apparatus, and instructions. At least part of the application programs may be downloaded from an external server through wireless communication.

Furthermore, the database 170 may be separate from the present device, or may be implemented as a memory connected by wire or wirelessly. In the case, the memory may include the type of a storage medium of at least one of a flash memory type, a hard disk type, a Solid State Disk (SSD) type, a Silicon Disk Drive (SDD) type, a multimedia card micro type, a memory of a card type (e.g., SD memory, XD memory, or the like), a random access memory (RAM), a static random access memory (SRAM), a read-only memory (ROM), an electrically erasable programmable read-only memory (EEPROM), a programmable read-only memory (PROM), a magnetic memory, a magnetic disk, and an optical disc.

Although not shown in FIG. 1, the preload measurement device 10 may include a Digital Signal Processor (DSP) including an amplifying means for amplifying a received heart sound (HS) and a filtering means for digitizing and simultaneously filtering the amplified heart sound. In this case, the filtering means may include an Analog-Digital Converter (ADC), a band-pass filter, or a high pass filter.

In an embodiment, the band-pass filter performs band-pass filtering on the digital signal of the converted heart sound. Because most of the noise capable of being collected by the esophageal stethoscope (P) is high frequency, a low frequency band of 8 to 1000 Hz may be used.

FIG. 2 is a flowchart illustrating a method for measuring a general anesthesia surgery patient preload based on acoustic variability index, according to an embodiment of the present disclosure. In the present disclosure, the method for measuring a general anesthesia surgery patient preload based on acoustic variability index may be understood as a technical methodology performed to measure a preload by detecting heart sound peak values by using Hilbert transform and Hilbert envelope for a heart sound based on acoustic variability index (AVI).

In operation S105, the preload measurement device 10 of FIG. 1 according to an embodiment of the present disclosure may receive heart-lung sound. According to an embodiment, the preload measurement device 10 may obtain the heart-lung sound of the patient from the heart sound detector 20 of FIG. 1.

In operation S110, the preload measurement device 10 according to an embodiment of the present disclosure may split a heart-lung sound. According to an embodiment, the preload measurement device 10 may split the heart-lung sound into a heart sound, which is a heartbeat sound, and a lung sound, which is a breathing sound in lungs.

Here, the heart sound (HS) is generated by valve movement due to the contraction and relaxation of atrium and ventricle and the dynamic movement of blood flow. In the past, heart sounds are obtained by using a stethoscope and are used as useful information for diagnosis, but it is subjective for each practitioner and has low accuracy.

To overcome these issues, the present disclosure obtains and utilizes PCG through the heart sound (HS). The PCG is obtained by displaying the heart sound (HS) in a visual form and is expressed as a graph including a frequency and amplitude. Accordingly, it is necessary to remove heart noise, which is a noise that is inevitably included in the heart sound (HS).

The PCG reflects cardiac function and hemodynamic status well. Moreover, the systolic time interval (STI) is closely related to a patient's preload due to the principle that "the more blood in the ventricles, the more time it takes to empty them".

The first heart sound S1 and the second heart sound S2 may be identified by analyzing the PCG, and the time difference between the first heart sound S1 and the second heart sound S2 (S1-S2 Time Interval: STI) is the systolic time interval (STI).

In operation S115, the preload measurement device 10 according to the embodiment of the present disclosure may perform a Hilbert transform on the heart sound and may fit Hilbert envelope on the transformed data. Because the Hilbert transform and the Hilbert envelope are well-known technical concepts, detailed descriptions will be omitted.

In operation S120, the preload measurement device 10 according to the embodiment of the present disclosure may detect the first heart sound S1 and the second heart sound S2 in the Hilbert envelope. According to an embodiment, the preload measurement device 10 may detect a heart sound based on the systolic time interval (STI). There are two main reasons for using the systolic time interval (STI) to measure the preload.

First, the first heart sound S1 may be heard in relation to the closure of an atrioventricular valve, because it includes the following four components. The primary contraction of ventricularly moving blood towards the atrium results in primary oscillations and closure of the atrioventricular valve. The secondary component is caused by the sudden strain of the closed atrioventricular valve. The tertiary component indicates the oscillations of blood flow between the aortic root and the ventricular wall. The quaternary component is associated with oscillations caused by the flow of blood into the medial side of the great vessels. The first heart sound includes sounds caused by the closure of the mitral valve between the left atrium and the left ventricle and the closure of the tricuspid valve between the right atrium and the right ventricle, and information related to the preload among these is the sound produced by the closure of the mitral valve.

Second, the second heart sound S2 represents the end of cardiac contraction and the beginning of cardiac relaxation, and may be heard at the time of closure of the aortic and pulmonary valves. The second heart sound S2 is the result of oscillations in the cardiovascular system caused by the deceleration and reversal of flow in the medial side of a pulmonary artery and an aorta. The second heart sound includes the sound generated by the closure of the aortic valve and pulmonary valve, and information related to the preload among these is the sound generated when the aortic valve is closed.

Accordingly, the systolic time interval (STI), which is the time difference between the first heart sound S1 and the second heart sound S2, becomes an index related to the preload. Because the blood needs to be discharged through the left ventricular outlet of the same area, it may be inferred that the more blood remains in the heart during the corresponding cardiac cycle, the longer the time required, and thus the longer the opening/closing time interval of two valves may be.

In operation S125, the preload measurement device 10 according to an embodiment of the present disclosure may detect the first heart sound S1 and the second heart sound S2 in the entire section of the heart sound. According to an embodiment, the preload measurement device 10 may obtain an integrated graph including an envelope by detecting the first heart sound S1 and the second heart sound S2 for the entire heart sound that is a measurement target. The integrated graph may be used to calculate the time interval of heart sounds based on peak values, a heart sound amplitude, and an area formed by heart sounds in the future.

In operation S130, the preload measurement device 10 according to an embodiment of the present disclosure may calculate at least one of a peak-specific location, amplitude, and an area for a heart sound waveform including an envelope. The peak-specific location, the amplitude, and the area of the heart sound waveform may be used to calculate the heart sound index.

In operation S135, the preload measurement device 10 according to an embodiment of the present disclosure may detect a breathing cycle from a separated lung sound. According to an embodiment, the preload measurement device 10 may split an expiratory time point and an inhalation time point by reflecting the expiratory characteristics and the inhalation characteristics from the lung sound, and may detect a breathing section, which is a time section between the expiratory and the inhalation. According to an embodiment, the preload measurement device 10 may specify inhalation and expiratory signals by using an inhalation-expiratory interval being shorter than an expiratory-inhalation interval.

In operation S140, the preload measurement device 10 according to an embodiment of the present disclosure may specify the breathing section. According to an embodiment, the preload measurement device 10 may calculate an average respiratory rate (PR) by averaging an inhalation-inhalation interval or an expiratory-expiratory interval, and may specify the breathing section by finding an inflection point through primary differentiation to specify an inhalation starting point.

In operation S145, the preload measurement device 10 according to an embodiment of the present disclosure may calculate a heart sound index for each breathing section. According to an embodiment, the preload measurement device 10 may use the amplitude of the second heart sound S2 as a heart sound index.

In operation S150, the preload measurement device 10 according to an embodiment of the present disclosure may output an acoustic variability index (AVI). According to an embodiment, the preload measurement device 10 may calculate the acoustic variability index through the calculation process of Equation 1.

[Equation 1]

$$AVI = a \times F(s)^{\alpha} \times G(s)^{\beta} \times H(s)^{\gamma}$$

Here, the first variable (F(s)) is defined based on Equation 2; the second variable (G(s)) is defined based on Equation 3; and, the third variable (H(s)) is defined based on Equation 4. Although only the first to third variables are described in the present disclosure, it is obvious to those skilled in the art that more heart rate-related indices may be considered and further calculated.

[Equation 2]

$$F(s) = \frac{STI_{\max} - STI_{\min}}{\langle STI \rangle}$$

[Equation 3]

$$G(s) = \frac{S1_{\mathrm{amp,max}} - S1_{\mathrm{amp,min}}}{\langle S1_{\mathrm{amp}} \rangle}$$

[Equation 4]

$$H(s) = \frac{S2_{\mathrm{amp}}}{\langle S2_{\mathrm{amp}} \rangle}$$

In other words, from some perspectives, it may be interpreted that the acoustic variability index (AVI) is related to the amount of ejection blood flow within heartbeats of a specific period.

The first variable (F(s)) may indicate how much the blood flow changes from a point in time when the valve opens when blood flows in, to a point in time when the valve closes when blood flows out. That is, the first variable may be interpreted as an index corresponding to blood flow fluctuations.

The second variable (G(s)) is related to a valve sound between the ventricle and the atrium and may generally be interpreted as an index corresponding to the myocardial contractility.

The third variable (H(s)) is the valve sound between the heart and arteries and may be generally interpreted as an index corresponding to vascular resistance.

In the present disclosure, the preload measurement device 10 is described as computing Equation 1, but is not limited thereto. For example, the preload measurement device 10 may calculate the acoustic variability index (AVI) derived as a result of combinations of various variables such as Equations 2 and 3, Equations 2 and 4, Equations 3 and 4, and Equations 2, 3 and 4, and thus may perform analysis on a heart-lung sound.

At least one component may be added or deleted to correspond to the performance of the components illustrated in FIG. 2. Furthermore, it will be easily understood by those skilled in the art that mutual locations of the components may be changed to correspond to the performance or structure of the system.

In the meantime, each component shown in FIG. 2 refers to software components and/or hardware components such as field programmable gate array (FPGA) and application specific integrated circuit (ASIC).

FIG. 3 is a diagram illustrating that a preload measurement device according to an embodiment of the present disclosure splits a heart sound and a lung sound from a heart-lung sound through a band-pass filter.

Referring to FIG. 3, a heart-lung sound may be split into a heart sound and a lung sound through a band pass filter (BPF). The preload measurement device 10 of FIG. 1 according to an embodiment of the present disclosure may split the heart sound and the lung sound by using the BPF provided in a DSP.

In an embodiment, a band of 25 to 100 Hz is a heart sound signal region including both a first heart sound signal and a second heart sound signal. In an embodiment, a band of 300 to 1800 Hz is a breathing sound signal region including both inhalation and expiratory signals. In an embodiment, a low frequency band of 25 Hz or less is noise caused by patient movement, and is the target region to be removed. In an embodiment, a band of 100 to 300 Hz is a signal processing interference region where a heart sound and a breathing sound are mixed, and is the target area to be removed.

Accordingly, the preload measurement device 10 according to an embodiment of the present disclosure may use a BPF that passes a band of 25 to 100 Hz and a band of 300 to 1800 Hz.

FIG. 4 is a waveform diagram showing that a heart sound is split from a heart-lung sound and transformed into an envelope form for analysis by a preload measurement device, according to an embodiment of the present disclosure. The heart-lung sound split module 120 of FIG. 1 may perform operations of FIG. 4.

Referring to an upper graph of FIG. 4, a heart-lung sound may be obtained by combining a heart sound and a lung sound, as indicated by a solid line graph and a dotted line graph. The heart-lung sound may be split into the heart sound and the lung sound by the methodology described in FIG. 4. According to FIG. 4, the heart sound split from the heart-lung sound is a part of the heart-lung sound, as indicated by the solid line graph.

The present disclosure generates a Hilbert envelope by performing Hilbert transform on the split heart sound (the solid line graph). Referring to a lower graph of FIG. 4, the schematic shape of the Hilbert envelope is illustrated.

FIG. 5 is a diagram for describing a method in which a preload measurement device according to an embodiment of the present disclosure determines a breathing cycle based on a peak. The breathing section specifying module 130 of FIG. 1 may perform the operation of FIG. 5.

The preload measurement device 10 according to an embodiment may perform a Hilbert transform on a lung sound (an upper graph of FIG. 5) and may generate an envelope. The preload measurement device 10 according to an embodiment may convert a peak value of respiration into a signal in a detectable format by applying a moving average to the envelope (a lower graph of FIG. 5).

The preload measurement device 10 according to an embodiment may detect a peak of a breathing signal by using local maxima. The preload measurement device 10 according to an embodiment may specify an inhalation signal and an expiratory signal by using an interval between inhalation (t2) and expiratory (t3) being shorter than an interval between expiratory (t3) and inhalation (t4).

The preload measurement device 10 according to an embodiment may calculate a respiratory rate (RR) as an average of inhalation-inhalation interval or expiratory-expiratory interval.

The preload measurement device 10 according to an embodiment may find an inflection point by performing primary differentiation on a graph forming an envelope, and may specify the detected inflection point as an inhalation starting point. As a result, the preload measurement device 10 may specify a breathing section P1.

FIG. 6 is a diagram showing a method, in which a preload measurement device according to an embodiment of the present disclosure obtains a heart sound index, and an error removal and split correction method. The heart sound index calculation module 140 of FIG. 1 may perform the operation of FIG. 6.

The preload measurement device 10 according to an embodiment may perform a Hilbert transform on a heart sound (a top graph of FIG. 6) and may generate an envelope. The preload measurement device 10 according to an embodiment may convert a peak value of the heart sound into a signal in a detectable format by applying a moving average to the envelope (the top graph of FIG. 6).

The preload measurement device 10 according to an embodiment may calculate a heart rate by applying a modified cross correlation method. According to an embodiment, the preload measurement device 10 may identify the peak of the first heart sound amplitude, which is generated initially, based on information about an interval between the first heart sound and a second heart sound, a shape, and a frequency. For example, the peak specific criteria are as follows.

(1) First heart sound peak-second heart sound time interval < second heart sound peak-first heart sound time interval
(2) First heart sound signal thickness > second heart sound signal thickness
(3) First heart sound signal frequency < second heart sound signal frequency

The preload measurement device 10 according to an embodiment may detect both peaks of the first heart sound signal and the second heart sound signal in an entire heart rate section (P2).

The preload measurement device 10 according to an embodiment may calculate a heart sound index associated with the peak amplitude, area, and time of each of the first heart sound signal and the second heart sound signal. The preload measurement device 10 may calculate a time interval between the first heart sound signal and second heart sound signal, a time interval variation between the first heart sound signal and second heart sound signal, an amplitude ratio between the first heart sound signal and second heart sound signal, and an amplitude variation between the first heart sound signal and second heart sound signal based on the heart sound index.

The preload measurement device 10 according to an embodiment may determine an error value by using information about an interval between the first heart sound signals, an interval between the second heart sound signals, the amplitude of each of the first heart sound signal and the second heart sound signal, an interval between the first heart sound signal and the second heart sound signal within the section P2. (The bottom graph of FIG. 6)

After removing the error value, the preload measurement device 10 according to an embodiment may recalculate a time interval between the first heart sound signal and the second heart sound signal, a time interval variation between the first heart sound signal and the second heart sound signal, an amplitude ratio between the first heart sound signal and the second heart sound signal, and an amplitude variation between the first heart sound signal and the second heart sound signal within one breathing section by correcting a removal section by using linear interpolation (the bottom graph of FIG. 6).

The preload measurement device 10 according to an embodiment may correct errors due to split in data indicating that a first heart sound split phenomenon (S1 Split) is prominent. FIGS. 7A and 7B are diagrams illustrating that a preload measurement device according to an embodiment of the present disclosure corrects a first heart sound split phenomenon.

Referring to FIG. 7A, STI and pulse pressure obtained within a specific section (P3) are compared with each other. FIG. 7B obtained by enlarging in detail the bottom graph of FIG. 7A is referenced. With reference to FIGS. 7A and 7B, it is described that the first heart sound signal is split and corrected.

A first heart sound signal is composed of the sound generated when a mitral valve and a tricuspid valve are closed, and STI needs to utilize a mitral valve signal, and thus it is difficult to measure the exact time interval when a tricuspid valve signal is determined as a peak.

Accordingly, the present disclosure may separate the mitral valve signal and the tricuspid valve signal by using curve fitting using a double Gaussian function. The preload measurement device 10 according to an embodiment may obtain only the mitral valve signal and may calculate a heart sound index such as STI.

FIG. 8 is a graph showing a comparison between the amplitude of a second heart sound and systemic vascular resistance (SVR) among heart sound indices obtained according to an embodiment of the present disclosure.

Referring to FIG. 8, the obtained second heart sound amplitude and the obtained SVR are compared with each other as shown in a graph. The black solid line in FIG. 8 indicates SVR, and the gray dotted line in FIG. 8 is a heart sound index, which is the amplitude of a second heart sound.

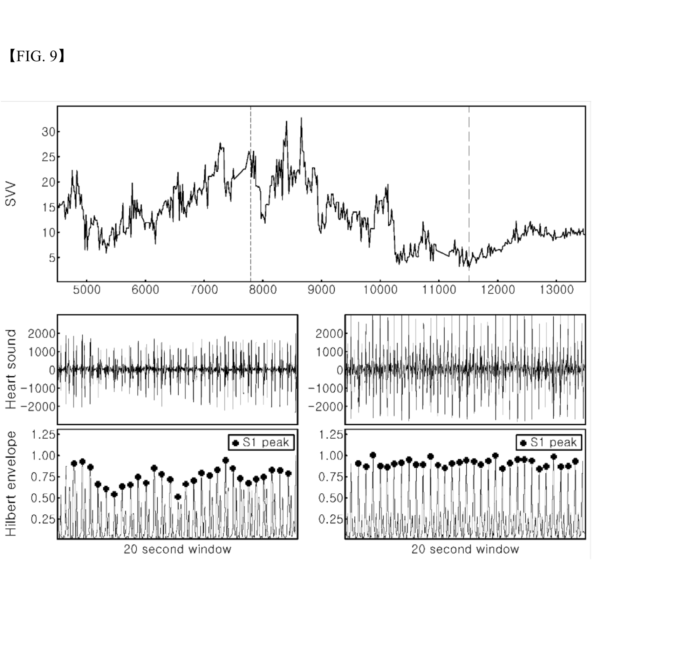

FIG. 9 is a graph showing a difference in a change amount of the first heart sound amplitude in a section (a dotted line area of the top graph) where SVV appears high, and a section (a dashed line area of the top graph) where SVV appears low.

Left and right graphs at the bottom of FIG. 9 represent a heart sound and an envelope in each of a region having high SVV, and a region having low SVV, and a cross-shaped marker at the bottom is the peak of the first heart sound amplitude in the corresponding region. The amount of change in a region having the high SVV is relatively large, and this may be used to understand Equation 3 in more detail.

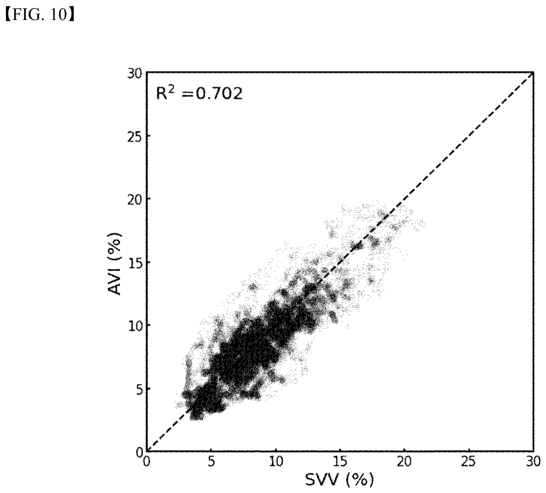

FIG. 10 is a graph showing a comparison between an acoustic variability index derived according to an embodiment of the present disclosure and SVV, which is one of existing utilized indices.

A horizontal axis of FIG. 10 represents SVV, and a vertical axis of FIG. 10 represents an acoustic variability index according to an embodiment of the present disclosure.

According to FIG. 10, the SVV and the acoustic variability index show the results of regression analysis. Regression analysis explanatory power $R^2$ is a value that indicates an extent to which a portion (SSR) explained by a regression line (Y=X) is occupied in the total variation (SST), and is calculated as "$R^2$ = SSR/SST = 1 - SSE/SST". That is, it is a numerical value indicating how well the equation of the regression line explains original data.

According to an embodiment of the present disclosure, statistical significance indicating that the SVV and the acoustic variability index are significantly similar to each other based on the fact that the regression analysis explanatory power $R^2$ is 0.702 was proven.

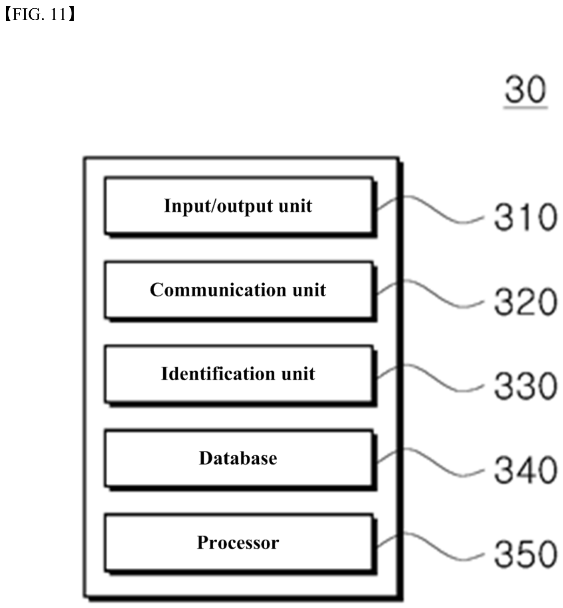

FIG. 11 is a block diagram illustrating an electronic device 30, which is an example of a preload measurement device, according to an embodiment of the present disclosure. The preload measurement device of FIG. 1 may be implemented with the electronic device 30 of FIG. 11.

Referring to FIG. 11, the electronic device 30 may include an input/output unit 310, a communication unit 320, an identification unit 330, a database 340, and a processor 350. Hereinafter, it is assumed that the electronic device 30 is an electronic device that implements a general anesthesia surgery patient preload measurement method based on an acoustic variability index, and the general anesthesia surgery patient preload measurement method based on an acoustic variability index is implemented through the electronic device 30.

The input/output unit 310 may be various interfaces or connection ports that receive a user input or output information to a user. The input/output unit 310 may be divided into an input module and an output module.

The input module receives the user input from the user. The input module may be used to enter image information (or signal), audio information (or signal), data, or information entered by a user. The input unit may include at least one of at least one camera, at least one microphone, and a user input unit. Speech data or image data collected by the input unit may be analyzed and processed as a control command of a user.

The user input may come in many forms, including a keystroke, a touch input, and a voice input. Examples of input modules capable of receiving the user input include touch sensors that detect a user's touch, microphones that receive voice signals, cameras that recognize gestures through image recognition, proximity sensors such as light sensors or infrared sensors that detect the user's approach, motion sensors that recognize the user's motion through acceleration sensors or gyro sensors, and various other input means that detect or receive various forms of user inputs, as well as traditional keypads, keyboards, and mice.

Here, the touch sensor may be implemented as a piezoelectric or electrostatic touch sensor that detects touch through a touch panel or touch film attached to a display panel, an optical touch sensor that detects touch by an optical method, or the like. Besides, the input module may also be implemented in the form of an input interface (USB port, PS/2 port, etc.) that connects an external input device that receives a user input instead of a device that detects a user input on its own.

The output module may output various pieces of information and may provide the pieces of information to the user. The output module is a comprehensive concept that includes a display that outputs images, a speaker (and/or an amplifier connected thereto) that outputs a sound, a haptic device that generates vibrations, and various other forms of output means. Besides, the output module may be implemented in the form of a port type of output interface that connects the individual output means described above.

For example, the output module in the form of a display may display text, still images, or video. The display is a concept that broadly refers to image display devices that include various forms of devices that may perform image output functions, such as liquid crystal displays (LCDs), light emitting diode (LED) displays, organic light emitting diode (OLED) displays, flat panel displays (FPDs), transparent displays, curved displays, flexible displays, 3D displays, holographic displays, projectors, and others. The display may be in the form of a touch display integrated with the touch sensor of the input module.

The communication unit 320 may communicate with an external device. Accordingly, the device may transmit and receive information with an external device through the communication unit. For example, the communication unit 320 may include at least one of a wired communication module, a wireless communication module, a short-range communication module, and a location information module.

Here, communication (i.e., sending and receiving data) may be done wired or wirelessly. To this end, the communication unit may be composed of a wired communication module that connects to the Internet, etc., via a Local Area Network (LAN), a mobile communication module that connects to a mobile communication network via a mobile communication base station to transmit and receive data, a short-range communication module that uses a Wireless Local Area Network (WLAN) series communication method such as Wi-Fi or a Wireless Personal Area Network (WPAN) series communication method such as Bluetooth or Zigbee, a satellite communication module that uses a Global Navigation Satellite System (GNSS) such as a Global Positioning System (GPS), or any combination thereof. The wireless communication technologies used for communication may include Narrowband Internet of Things (NB-IoT) for low-power communication. In this case, for example, the NB-IoT technology may be an example of a Low Power Wide Area Network (LPWAN) technology and may be implemented with standards such as LTE Cat (category) NB1 and/or LTE Cat NB2, and is not limited to the above-described names. Additionally or alternatively, the wireless communication technology implemented in a wireless device according to various embodiments may perform communication based on LTE-M technology. At this time, for example, LTE-M technology may be an example of LPWAN technology and may be referred to as various names such as enhanced Machine Type Communication (eMTC). For example, the LTE-M technology may be implemented by at least one of various standards, such as 1) LTE CAT 0, 2) LTE Cat M1, 3) LTE Cat M2, 4) LTE non-BL (non-Bandwidth Limited), 5) LTE-MTC, 6) LTE Machine Type Communication, and/or 7) LTE M, and is not limited to the above-mentioned names. Additionally or alternatively, the wireless communication technology implemented in the wireless device according to various embodiments may include at least one of ZigBee, Bluetooth, and Low Power Wide Area Network (LPWAN) considering low-power communication, and is not limited to the above-described names. For example, the ZigBee technology may create personal area networks (PANs) for small/low-power digital communications based on various standards such as IEEE 802.15.4, and may be referred to as various names.

Here, in addition to various wired communication modules such as a Local Area Network (LAN) module, a Wide Area Network (WAN) module, or a Value Added Network (VAN) module, the wired communication module may include a variety of cable communication modules such as Universal Serial Bus (USB), High Definition Multimedia Interface (HDMI), Digital Visual Interface (DVI), recommended standard232 (RS-232), power line communication, or plain old telephone service (POTS).

Here, the wireless communication module may include a wireless communication module for supporting various wireless communication methods such as Global System for Mobile (GSM) communication, Code Division Multiple Access (CDMA), Wideband Code Division Multiple Access (WCDMA), Universal Mobile Telecommunication System (UMTS), Time Division Multiple Access (TDMA), Long Term Evolution (LTE), 4G, 5G, and 6G in addition to a Wi-Fi module and Wireless broadband module.

The wireless communication module may include a wireless communication interface including an antenna and a transmitter that transmit signals. Moreover, the wireless communication module may further include a signal conversion module that modulates a digital control signal, which is output from the controller through a wireless communication interface, into an analog wireless signal under the control of a controller.

The wireless communication module may include a wireless communication interface including an antenna and a receiver that receive a signal. Furthermore, the wireless communication module may further include a signal conversion module for demodulating an analog wireless signal, which is received through the wireless communication interface, into a digital control signal.

The short-range communication module may be used for short range communication, and may support short-range communication by using at least one of Bluetooth™, radio frequency identification (RFID), infrared data association (IrDA), ultra wideband (UWB), ZigBee, near field communication (NFC), Wireless-Fidelity (Wi-Fi), Wi-Fi Direct, and wireless universal serial bus (Wireless USB) technologies.

The location information module is a module for obtaining a location (or current location) of an electronic device according to an embodiment of the present disclosure, and representative examples thereof include a Global Positioning System (GPS) module or a Wireless Fidelity (Wi-Fi) module. For example, when the GPS module is utilized, the location of the electronic device may be obtained by using signals sent from GPS satellites. For another example, when the Wi-Fi module is utilized, the location of this device may be obtained based on information from the Wi-Fi module and the wireless Access Point (AP) that transmits or receives wireless signals. As required, the location information module may perform a function of any other module of the communication unit to obtain data associated with the location of the present device, alternatively or additionally. The location information module is a module used to obtain the location (or current location) of the present device, and is not limited to a module that directly calculates or obtains the location of the present device.

The identification unit 330 may be a comprehensive concept including a camera that recognizes objects through image recognition, a detection sensor that detects approaching objects, a touch sensor according to a user input, and various forms of identification/sensing means that detect or receive various forms of external inputs.

The identification unit may be understood as being identical to the input module within the input/output unit 310 and/or may be understood as being separate from the input module. The identification unit 330 may further include, but is not limited to, one or more of a magnetic sensor, an acceleration sensor, a temperature/humidity sensor, an infrared sensor, a gyroscope sensor, a position sensor (e.g., GPS), a barometric pressure sensor, a proximity sensor, an RGB sensor (illuminance sensor), a radar sensor, an illumination sensor, and a current sensor. Because the function of each sensor may be intuitively inferred by those skilled in the art from their names, a detailed description will be omitted.

The database 340 may store various pieces of information. The database may store data temporarily or semi-permanently. For example, the database may store operating programs (OS) for operating a first device and/or a second device, data associated with hosting a website, data associated with a program or application (e.g., a web application) for generating braille, or the like. Moreover, the database may store modules in the form of computer code as described above.

Examples of the database 340 include hard disk drives (HDDs), solid state drives (SSDs), flash memory, read-only memory (ROMs), and random access memory (RAMs). The database may be provided as a built-in or removable type.

The processor 350 controls overall operations of the electronic device 30. To this end, the processor 350 may perform computation and processing of various pieces of information and may control the operations of components of the first device and/or the second device. For example, the processor 350 may execute a program or an application for measuring a general anesthesia surgery patient preload based on an acoustic variability index.

The processor 350 may be implemented as a computer or a device similar thereto by using hardware, software, or any combination thereof. In terms of hardware, the processor 350 may be provided in the form of an electronic circuit that processes electrical signals and performs control functions. In terms of software, the processor 350 may be provided in the form of a program that operates a hardware processor. In the meantime, unless otherwise specifically stated in the description below, the operations of the first device and/or the second device may be interpreted as being performed under the control of the processor 350. That is, when modules implemented as programs or applications for measuring a general anesthesia surgery patient preload based on an acoustic variability index are executed, the modules may be interpreted as allowing the processor 350 to perform the following operations on the first device and/or the second device.

The processor 350 may be implemented with a memory that stores data regarding an algorithm for controlling operations of components within the present device, or a program for implementing the algorithm, and the at least one processor (not illustrated) that perform the above-described operation by using the data stored in the memory. At this time, the memory and the processor may be implemented as separate chips. Alternatively, the memory and the processor may be implemented as a single chip.

Furthermore, the processor 350 may control one of the components described above or the combination of the components to implement various embodiments of the present disclosure described below with reference to FIGS. 1 to 8 on the present disclosure.

In an embodiment, the electronic device 30 includes a memory that stores at least one instruction and a processor that performs a preload measurement function by executing the instruction. The processor splits a heart sound of the patient through a high-pass filter and a band-pass filter by using a heart-lung sound split module, specifies a section between inhalation and expiratory of the patient by using an envelope of a waveform generated through a Hilbert transform by using a breathing section specifying module, detects a first heart sound signal and a second heart sound signal corresponding to local maxima of the envelope by using a heart sound index calculation module, and calculates an acoustic variability index from at least one of a time variation, an amplitude variation, and an area variation of each of the first heart sound signal and the second heart sound signal by using an acoustic variability index computation module.

In the meantime, each component shown in FIG. 11 refers to software components and/or hardware components such as field programmable gate array (FPGA) and application specific integrated circuit (ASIC).

In summary, the present disclosure may be implemented through various means. For example, various embodiments may be implemented by hardware, firmware, software, or any combination thereof.

In the case of implementation by hardware, a method according to an embodiment of the present disclosure may be implemented by one or more application specific integrated circuits (ASICs), digital signal processors (DSPs), digital signal processing devices (DSPDs), programmable logic devices (PLDs), field programmable gate arrays (FPGAs), processors, controllers, microcontrollers, microprocessor, and the like.

In the case of implementation by firmware or software, the method according to an embodiment of the present disclosure may be implemented in the form of a module, procedure, or function that performs the functions or operations described above. For example, a software code may be stored in a memory to be run by a processor. The memory may be located inside or outside the processor, and may exchange data with the processor by various means known in the art.

Steps or operations of the method or algorithm described with regard to an embodiment of the present disclosure may be implemented directly in hardware, may be implemented with a software module executable by hardware, or may be implemented by a combination thereof. The software module may reside in a random access memory (RAM), a read only memory (ROM), an erasable programmable ROM (EPROM), an electrically erasable programmable ROM (EEPROM), a flash memory, a hard disk, a removable disk, a CD-ROM, or a computer-readable recording medium well known in the art to which the present disclosure pertains.

Meanwhile, the disclosed embodiments may be implemented in a form of a recording medium storing instructions executable by a computer. The instructions may be stored in a form of program codes, and, when executed by a processor, generate a program module to perform operations of the disclosed embodiments. The recording medium may be implemented as a computer-readable recording medium.

The computer-readable recording medium may include all kinds of recording media in which instructions capable of being decoded by a computer are stored. For example, there may be read only memory (ROM), random access memory (RAM), magnetic tape, magnetic disk, flash memory, optical data storage device, and the like.

## Claims

1. A method for analyzing a heart sound by an electronic device for measuring a preload of a general anesthesia surgery patient, the method comprising:

   splitting a heart sound and a breathing sound from heart-lung sound data of a patient obtained externally;
   specifying a breathing section from the split breathing sound;
   obtaining a heart sound envelope for obtaining a peak signal by converting a heart sound signal;
   obtaining a heart sound index from the heart sound envelope; and
   calculating an acoustic variability index (AVI) by analyzing the heart sound index.

2. The method of claim 1, wherein the splitting of the heart sound and the breathing sound includes:

   removing a signal caused by movement of the patient by using a high-pass filter; and
   splitting the heart sound and the breathing sound by using a band-pass filter.

3. The method of claim 1, wherein the specifying of the breathing section includes:

   specifying locations of inhalation and expiratory by using a Hilbert transform and a Hilbert envelope; and
   specifying a time section from the inhalation to the expiratory as the breathing section based on the locations of the inhalation and the expiratory.

4. The method of claim 1, wherein the obtaining of the heart sound envelope includes:

   obtaining an envelope of a waveform generated as a result of performing a Hilbert transform on the heart sound;
   flattening the envelope; and
   removing noise from the flattened envelope.

5. The method of claim 1, wherein the obtaining of the heart sound envelope further includes:

   specifying a first heart sound signal and a second heart sound signal based on local maxima in an initial input section of the heart sound envelope;
   detecting a peak of each of the first heart sound signal and the second heart sound signal based on modified auto-correlation in an entire input section of the heart sound envelope;
   re-specifying a location of the peak based on the first heart sound signal thus split;
   removing a time section including an undetected peak;
   performing linear interpolation on the removed time section; and
   obtaining at least one of amplitude, a location, and an area of each of the first heart sound signal and the second heart sound signal.

6. The method of claim 1, wherein the calculating of the AVI includes:

measuring a time variation between a first heart sound signal and a second heart sound signal from the heart sound index;
measuring an amplitude variation and an area variation of the first heart sound signal;
measuring an amplitude variation and an area variation of the second heart sound signal;
calculating the acoustic variability index from at least one of the time variation, the amplitude variation, and the area variation.

7. A method for analyzing a heart sound by an electronic device for measuring a preload of a general anesthesia surgery patient, the method comprising:

splitting a heart sound and a breathing sound from heart-lung sound data of a patient obtained externally;
obtaining an envelope of a waveform generated as a result of performing a Hilbert transform on the heart sound;
detecting a first heart sound signal and a second heart sound signal;
calculating at least one of a peak-specific location, amplitude, and an area of the waveform;
detecting a breathing cycle from the breathing sound;
specifying a breathing section from the breathing cycle;
calculating a heart sound index for the respective breathing section; and
calculating an AVI by analyzing the heart sound index.

8. The method of claim 7, wherein the splitting of the heart sound and the breathing sound includes:

removing a signal caused by movement of the patient by using a high-pass filter; and
splitting the heart sound and the breathing sound by using a band-pass filter.

9. The method of claim 7, wherein the specifying of the breathing section includes:
specifying locations of inhalation and expiratory by using a Hilbert envelope.

10. The method of claim 7, wherein the obtaining of the envelope of the waveform includes:

flattening the envelope; and
removing noise from the flattened envelope.

11. The method of claim 7, wherein the obtaining of the heart sound envelope further includes:

specifying a first heart sound signal and a second heart sound signal based on local maxima in an initial input section of the envelope;
detecting a peak of each of the first heart sound signal and the second heart sound signal based on modified auto-correlation in an entire input section of the envelope;
re-specifying a location of the peak based on the first heart sound signal thus split;

12. The method of claim 11, wherein the obtaining of the heart sound envelope further includes:

removing a time section including an undetected peak;
performing linear interpolation on the removed time section; and
obtaining at least one of amplitude, a location, and an area of each of the first heart sound signal and the second heart sound signal.

13. The method of claim 7, wherein the calculating of the AVI includes:

measuring a time variation between the first heart sound signal and the second heart sound signal from the heart sound index;
measuring an amplitude variation and an area variation of the first heart sound signal;
measuring an amplitude variation and an area variation of the second heart sound signal;
calculating the acoustic variability index from at least one of the time variation, the amplitude variation, and the area variation.

14. An electronic device for measuring a preload of a general anesthesia surgery patient, the electronic device comprising:

a memory configured to store at least one instruction; and
a processor configured to perform a preload measurement function by executing the instruction,
wherein the processor is configured to:

split a heart sound of the patient through a high-pass filter and a band-pass filter by using a heart-lung sound split module;
specify a section between inhalation and expiratory of the patient by using an envelope of a waveform generated through a Hilbert transform by using a breathing section specifying module;
detect a first heart sound signal and a second heart sound signal corresponding to local maxima of the envelope by using a heart sound index calculation module; and
calculate an acoustic variability index from at least one of a time variation, an amplitude variation, and an area variation of each of the first heart sound signal and the second heart sound signal by using an acoustic variability index computation module.

15. The electronic device of claim 14, wherein the high-pass filter removes a signal caused by movement of the patient, and
wherein the band-pass filter splits a heart sound and a breathing sound.

【FIG. 1】

【FIG. 2】

【FIG. 3】

【FIG. 4】

【FIG. 5】

【FIG. 6】

【FIG. 7A】

【FIG. 7B】

【FIG. 8】

【FIG. 9】

【FIG. 10】

【FIG. 11】

30

| Input/output unit | 310 |
| Communication unit | 320 |
| Identification unit | 330 |
| Database | 340 |
| Processor | 350 |

## INTERNATIONAL SEARCH REPORT

| | International application No. |
|---|---|
| | **PCT/KR2023/020660** |

**A.    CLASSIFICATION OF SUBJECT MATTER**

**A61B 5/02**(2006.01)i; **A61B 7/02**(2006.01)i; **G16H 50/20**(2018.01)i

According to International Patent Classification (IPC) or to both national classification and IPC

**B.    FIELDS SEARCHED**

Minimum documentation searched (classification system followed by classification symbols)

A61B 5/02(2006.01); A61B 5/0402(2006.01); A61B 5/08(2006.01); A61M 16/10(2006.01); G16H 20/17(2018.01); G16H 50/70(2018.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 심음(heart sound), 호흡음(respiratory sound), 음향가변성지표(acoustic variabilit y index), 포락선(envelope)

**C.    DOCUMENTS CONSIDERED TO BE RELEVANT**

| Category* | Citation of document, with indication, where appropriate, of the relevant passages | Relevant to claim No. |
|---|---|---|
| A | KR 10-2020-0104341 A (RESMED SENSOR TECHNOLOGIES LIMITED) 03 September 2020 (2020-09-03)<br>See paragraphs [0038]-[0042], [0071], [0165]-[0166] and [0208]; claims 46, 51, 57 and 69-70; and figures 1-16. | 1-15 |
| A | KR 10-2006-0088770 A (SAMSUNG ELECTRONICS CO., LTD.) 07 August 2006 (2006-08-07)<br>See paragraphs [0015] and [0026]-[0027]; and figures 3-7. | 1-15 |
| A | US 2012-0132202 A1 (BURTON, David et al.) 31 May 2012 (2012-05-31)<br>See paragraphs [0009]-[0017]. | 1-15 |
| A | WO 2006-137067 A2 (EARLYSENSE LTD.) 28 December 2006 (2006-12-28)<br>See page 9, line 13 – page 11, line 23. | 1-15 |
| A | KR 10-2021-0023133 A (IUCF-HYU (INDUSTRY-UNIVERSITY COOPERATION FOUNDATION HANYANG UNIVERSITY)) 04 March 2021 (2021-03-04)<br>See paragraphs [0024]-[0053]; and figures 1-2. | 1-15 |

☐ Further documents are listed in the continuation of Box C.    ☑ See patent family annex.

| | |
|---|---|
| *    Special categories of cited documents:<br>"A"  document defining the general state of the art which is not considered to be of particular relevance<br>"D"  document cited by the applicant in the international application<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
|---|---|
| **18 March 2024** | **18 March 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT
### Information on patent family members

International application No.

**PCT/KR2023/020660**

| Patent document cited in search report | | | Publication date (day/month/year) | Patent family member(s) | | | Publication date (day/month/year) |
|---|---|---|---|---|---|---|---|
| KR | 10-2020-0104341 | A | 03 September 2020 | CN | 111655125 | A | 11 September 2020 |
| | | | | CN | 111655125 | B | 18 August 2023 |
| | | | | EP | 3727134 | A1 | 28 October 2020 |
| | | | | EP | 3727134 | B1 | 25 January 2023 |
| | | | | JP | 2021-508279 | A | 04 March 2021 |
| | | | | JP | 7202385 | B2 | 11 January 2023 |
| | | | | US | 2020-0367810 | A1 | 26 November 2020 |
| | | | | WO | 2019-122412 | A1 | 27 June 2019 |
| KR | 10-2006-0088770 | A | 07 August 2006 | US | 2006-0173373 | A1 | 03 August 2006 |
| | | | | US | 7930886 | B2 | 26 April 2011 |
| US | 2012-0132202 | A1 | 31 May 2012 | CN | 1713850 | A | 28 December 2005 |
| | | | | CN | 1713850 | B | 18 April 2012 |
| | | | | CN | 1713850 | C | 28 December 2005 |
| | | | | EP | 1551286 | A2 | 13 July 2005 |
| | | | | EP | 1551286 | A4 | 24 June 2009 |
| | | | | EP | 1551286 | B1 | 22 November 2017 |
| | | | | JP | 2006-516100 | A | 22 June 2006 |
| | | | | JP | 2010-104814 | A | 13 May 2010 |
| | | | | JP | 4865229 | B2 | 01 February 2012 |
| | | | | KR | 10-1060923 | B1 | 30 August 2011 |
| | | | | KR | 10-1177207 | B1 | 24 August 2012 |
| | | | | KR | 10-1309971 | B1 | 17 September 2013 |
| | | | | KR | 10-1318718 | B1 | 16 October 2013 |
| | | | | KR | 10-2005-0072435 | A | 11 July 2005 |
| | | | | KR | 10-2010-0095662 | A | 31 August 2010 |
| | | | | KR | 10-2010-0097764 | A | 03 September 2010 |
| | | | | KR | 10-2010-0097765 | A | 03 September 2010 |
| | | | | KR | 10-2010-0097766 | A | 03 September 2010 |
| | | | | KR | 10-2012-0094156 | A | 23 August 2012 |
| | | | | US | 2005-0217674 | A1 | 06 October 2005 |
| | | | | US | 2012-0130205 | A1 | 24 May 2012 |
| | | | | US | 2012-0136405 | A1 | 31 May 2012 |
| | | | | US | 8069852 | B2 | 06 December 2011 |
| | | | | WO | 2004-032719 | A2 | 22 April 2004 |
| | | | | WO | 2004-032719 | A3 | 10 June 2004 |
| WO | 2006-137067 | A2 | 28 December 2006 | CN | 101365373 | A | 11 February 2009 |
| | | | | EP | 1786315 | A2 | 23 May 2007 |
| | | | | EP | 1786315 | A4 | 03 March 2010 |
| | | | | EP | 1898777 | A2 | 19 March 2008 |
| | | | | EP | 1898777 | A4 | 03 March 2010 |
| | | | | EP | 1955233 | A2 | 13 August 2008 |
| | | | | EP | 1955233 | A4 | 11 November 2009 |
| | | | | EP | 2505131 | A1 | 03 October 2012 |
| | | | | JP | 2007-537777 | A | 27 December 2007 |
| | | | | JP | 2009-501557 | A | 22 January 2009 |
| | | | | JP | 2009-532072 | A | 10 September 2009 |
| | | | | JP | 2013-154190 | A | 15 August 2013 |
| | | | | JP | 4809779 | B2 | 09 November 2011 |
| | | | | JP | 5155856 | B2 | 06 March 2013 |
| | | | | JP | 5281406 | B2 | 04 September 2013 |

Form PCT/ISA/210 (patent family annex) (July 2022)

## INTERNATIONAL SEARCH REPORT
### Information on patent family members

| International application No. |
|---|
| **PCT/KR2023/020660** |

| Patent document cited in search report | Publication date (day/month/year) | Patent family member(s) | | | Publication date (day/month/year) |
|---|---|---|---|---|---|
| | | KR | 10-1182994 | B1 | 19 September 2012 |
| | | KR | 10-2008-0034437 | A | 21 April 2008 |
| | | US | 10194810 | B2 | 05 February 2019 |
| | | US | 10939829 | B2 | 09 March 2021 |
| | | US | 2005-0192508 | A1 | 01 September 2005 |
| | | US | 2006-0170501 | A1 | 03 August 2006 |
| | | US | 2006-0224076 | A1 | 05 October 2006 |
| | | US | 2006-0241510 | A1 | 26 October 2006 |
| | | US | 2007-0118054 | A1 | 24 May 2007 |
| | | US | 2008-0114260 | A1 | 15 May 2008 |
| | | US | 2008-0269625 | A1 | 30 October 2008 |
| | | US | 2011-0015535 | A1 | 20 January 2011 |
| | | US | 2011-0282216 | A1 | 17 November 2011 |
| | | US | 2013-0137998 | A1 | 30 May 2013 |
| | | US | 2013-0144178 | A1 | 06 June 2013 |
| | | US | 2013-0245502 | A1 | 19 September 2013 |
| | | US | 2013-0281866 | A1 | 24 October 2013 |
| | | US | 2014-0012099 | A1 | 09 January 2014 |
| | | US | 2014-0171816 | A1 | 19 June 2014 |
| | | US | 2014-0207204 | A1 | 24 July 2014 |
| | | US | 2014-0350360 | A1 | 27 November 2014 |
| | | US | 2015-0087932 | A1 | 26 March 2015 |
| | | US | 2015-0164433 | A1 | 18 June 2015 |
| | | US | 2015-0190087 | A1 | 09 July 2015 |
| | | US | 2015-0327792 | A1 | 19 November 2015 |
| | | US | 2016-0120466 | A1 | 05 May 2016 |
| | | US | 2017-0281017 | A1 | 05 October 2017 |
| | | US | 2019-0183353 | A1 | 20 June 2019 |
| | | US | 2021-0212572 | A1 | 15 July 2021 |
| | | US | 7077810 | B2 | 18 July 2006 |
| | | US | 7279978 | B2 | 09 October 2007 |
| | | US | 7314451 | B2 | 01 January 2008 |
| | | US | 8376954 | B2 | 19 February 2013 |
| | | US | 8403865 | B2 | 26 March 2013 |
| | | US | 8491492 | B2 | 23 July 2013 |
| | | US | 8517953 | B2 | 27 August 2013 |
| | | US | 8603010 | B2 | 10 December 2013 |
| | | US | 8679030 | B2 | 25 March 2014 |
| | | US | 8679034 | B2 | 25 March 2014 |
| | | US | 8731646 | B2 | 20 May 2014 |
| | | US | 8840564 | B2 | 23 September 2014 |
| | | US | 8942779 | B2 | 27 January 2015 |
| | | US | 8992434 | B2 | 31 March 2015 |
| | | US | 9026199 | B2 | 05 May 2015 |
| | | US | 9131891 | B2 | 15 September 2015 |
| | | US | 9131902 | B2 | 15 September 2015 |
| | | US | 9265445 | B2 | 23 February 2016 |
| | | US | 9681838 | B2 | 20 June 2017 |
| | | WO | 2005-074361 | A2 | 18 August 2005 |
| | | WO | 2005-074361 | A3 | 23 April 2009 |

Form PCT/ISA/210 (patent family annex) (July 2022)

## INTERNATIONAL SEARCH REPORT
### Information on patent family members

| International application No. |
|---|
| **PCT/KR2023/020660** |

| Patent document cited in search report | | Publication date (day/month/year) | Patent family member(s) | | | Publication date (day/month/year) |
|---|---|---|---|---|---|---|
| | | | WO | 2006-137067 | A3 | 27 September 2007 |
| | | | WO | 2007-052108 | A2 | 10 May 2007 |
| | | | WO | 2007-052108 | A3 | 16 April 2009 |
| KR | 10-2021-0023133 A | 04 March 2021 | KR | 10-2304714 | B1 | 24 September 2021 |

Form PCT/ISA/210 (patent family annex) (July 2022)

**REFERENCES CITED IN THE DESCRIPTION**

**Non-patent literature cited in the description**

- **JUNGYO SUH** ; **SANGWOOK LEE**. Preoperative prediction of the need for arterial and central venous catheterization using machine learning techniques. *Scientific reports*, 2022